# EUROPÄISCHE PATENTSCHRIFT

(11) **EP 1 910 710 B1**
(45) Veröffentlichungstag und Bekanntmachung des Hinweises auf die Patenterteilung: **19.10.2011**
(21) Anmeldenummer: 06776180.9
(22) Anmeldetag: 11.07.2006
(51) Int. Cl.: F16H 7/08, F16H 7/12

(54) **DÄMPFUNGSVORRICHTUNG FÜR EINEN ZUGMITTELTRIEB**
DAMPING DEVICE FOR A TRACTION MECHANISM DRIVE
DISPOSITIF D'AMORTISSEMENT POUR UNE TRANSMISSION PAR ELEMENT FLEXIBLE

(30) Priorität: 22.07.2005 DE 102005034332
(43) Veröffentlichungstag der Anmeldung: 16.04.2008
(73) Patentinhaber: Schaeffler Technologies GmbH & Co. KG, 91074 Herzogenaurach (DE)
(72) Erfinder: HARTMANN, Bernd, 91085 Weisendorf (DE); KERN, Roman, 91301 Forchheim (DE)
(86) Internationale Anmeldenummer: PCT/EP2006/006749
(87) Internationale Veröffentlichungsnummer: WO 2007/009631

(56) Entgegenhaltungen:
- WO-A-03/089809
- WO-A-2005/059402
- DE-A1- 10 360 288
- DE-U1- 9 006 213
- US-A- 4 822 008

## Beschreibung

### Gebiet der Erfindung

Die Erfindung bezieht sich auf eine Dämpfungsvorrichtung für einen Zugmitteltrieb, bei der sich ein um eine Drehachse schwenkbarer Spannarm zur Erzeugung und Aufrechterhaltung einer Vorspannung eines Zugmittels über eine Spannrolle an dem Zugmittel mit Hilfe von mechanischen Dämpfungsmitteln dämpfend abstützt.

### Hintergrund der Erfindung

Derartige, beispielsweise aus der DE 100 21 708 A1, der EP 0779 452 A2 und der DE 695 11 656 T2 bekannte Dämpfungsvorrichtungen, weisen wenigstens ein als ein Reibungselement ausgebildetes Gleitlager auf, das koaxial an der Drehachse angeordnet ist und in Wirkverbindung mit wenigstens einer ebenfalls koaxial angeordneten Feder steht. Diese Feder übt eine in Umfangsrichtung des Zugmitteltriebes wirkende Kraftkomponente aus, die den Spannarm federnd, in Verbindung mit einer an dem freien Ende des Spannarms drehbar gelagerten Spannrolle, an dem Zugmittel abstützt. Die Feder ist vorteilhaft, wie beispielsweise in der DE 101 52 364 A1 beschrieben, zur Bauraumoptimierung als eine konisch gewickelte Schraubendrehfeder ausgebildet. Entsprechend ist das Reibungselement vorzugsweise als eine konische Reibungsbuche ausgebildet, die, bei durch Stöße oder Schwingungen hervorgerufenen Schwenkbewegungen des Spannarms, dämpfend wirkt. Das Drehlager ist an einem ortsfesten Maschinenteil, beispielsweise an einem Kurbelgehäuse eines Verbrennungsmotors in einem Kraftfahrzeug befestigt, und der zugehörige Zugmitteltrieb dient beispielsweise zum Antrieb eines oder mehrerer Nebenaggregate (Generator, Wasserpumpe, Ventilator, etc.) des Kraftfahrzeuges.

Die derartigen auch als Konusspanner bekannten Spann- und Dämpfungssysteme haben sich bereits in der Praxis an sich bewährt. Nachteilig daran wirkt sich jedoch aus, dass die Dämpfungseinheiten sowohl funktionell als auch geometrisch an das Drehlager des Schwenkarms gekoppelt sind. Dadurch sind die konstruktiven Möglichkeiten bei der Konzeption des Spannsystems relativ eingeschränkt. Zudem wird insbesondere das Drehlager im Betrieb starken Schwingungs- und Stoßbelastungen ausgesetzt. Der Spannarm erfordert daher, zur Gewährleistung einer hohen Lebensdauer, einen relativ hohen Konstruktions- und Kostenaufwand. Schließlich wird der Querschnitt des Spannarms im Wesentlichen durch die Länge des Dämpfungssystems bestimmt und erfordert trotz konischer Feder- bzw. Reibelemente eine relativ große Bautiefe.

Aus dem WO 03/089809 ist eine Dämpfungsvorrichtung gemäß dem oberbegriff des Anspruchs 1 bekannt.

### Aufgabe der Erfindung

Der Erfindung liegt die Aufgabe zugrunde, eine Dämpfungsvorrichtung an einem Spannarm eines Zugmitteltriebes zu schaffen, die mit geringerem Kostenund Konstruktionsaufwand herstellbar ist, die effektiv und verschleißarm im Betrieb ist, und die mit einem geringen Bauraum, insbesondere mit einer verringerten Bautiefe des Spannarms realisierbar ist.

### Zusammenfassung der Erfindung

Der Erfindung liegt die Erkenntnis zugrunde, dass unter Ausnutzung einer Abstützung an einem Maschinenteil, mit einer von der Drehachse des Spannarms unabhängig konstruierten Dämpfungseinheit, die an einer beliebig vorgebbaren Stelle des Spannarms positionierbar ist, eine kostengünstige und bauraumsparende Möglichkeit zur Realisierung einer sehr effizienten mechanischen Dämpfung in Zugmitteltrieben geschaffen werden kann.

Die Erfindung geht gemäß den Merkmalen des Anspruchs 1 daher aus von einer Dämpfungsvorrichtung für einen Zugmitteltrieb, bei der sich ein um eine Drehachse schwenkbarer Spannarm zur Erzeugung und Aufrechterhaltung einer Vorspannung eine Zugmittels über eine Spannrolle an dem Zugmittel mit Hilfe von mechanischen Dämpfungsmitteln dämpfend abstützt. Zudem ist vorgesehen, dass in den Spannarm ein quer zur Drehachse beabstandeter Reibungsdämpfer integriert ist, mit einem seitlich aus dem Spannarm vorstehenden Kolben, dessen Ende sich stirnseitig an einem ortsfesten Maschinenteil abstützt, mit Mitteln zur Kompensation eines durch Schwenkbewegungen des Spannarms entstehenden Versatzes zwischen dem Maschinenteil und dem Spannarm, und mit einem in einem Aufnahmeraum innerhalb des Spannarms angeordneten Reibkörper, über den Schwenkbewegungen des Spannarms durch eine damit verbundene Gleitreibung zwischen dem Reibkörper und einer Reibfläche dämpfbar sind.

Durch diesen Aufbau wird vorteilhaft erreicht, dass der Spannarm direkt an einem ortsfesten Bauteil, beispielsweise an einem Verbrennungsmotor abgestützt und gedämpft wird. Die Abstützung erfolgt über einen Kolben, wobei die Kontaktgeometrie so gewählt ist, dass die Krafteinleitung stets senkrecht zur Kolbenstirnfläche erfolgt. Dazu sind Kompensationsmittel vorgesehen, die den entstehenden Versatz zwischen Kolben und Spannarm bei dessen durch Stöße und/oder Schwingungen verursachten Schwenkbewegungen kompensieren. Dadurch wird das Drehlager bei Schwenkbewegungen des Arms entlastet. Das Drehlager kann dadurch konstruktiv einfach ausgeführt sein.

Der Reibungsdämpfer ist quer zur Drehachse in den Hebelarm eingebaut. Damit wird eine sehr geringe Bautiefe des Hebelarms ermöglicht, was besonders im Kraftfahrzeugbereich, bei einem relativ geringen zur Verfügung stehenden Bauraum im Motorraum, von zunehmender Bedeutung ist.

Außerdem kann vorgesehen sein, dass der Reibkörper als ein mit seiner Grundfläche in Richtung des Maschinenteils ausgerichteter Kegelstumpf ausgebildet ist, der in einem in dem Aufnahmeraum gehaltenen und als ein Gleitkörper ausgebildeten ringförmigen Kegelsitz steckt, der mit Hilfe eines Federelementes formschlüssig gegen den Kegelstumpf gehalten wird, wobei eine Berührfläche zwischen dem Kegelstumpf und dem Kegelsitz die Reibfläche bildet.

Dabei ist vorzugsweise vorgesehen, dass die Versatzkompensationsmittel als eine Abrundung an der zur dem Maschinenteil benachbarten Stirnfläche des Kolbens und als eine zu der Abrundung als eine Gegenfläche ausgebildete Kontaktfläche an dem Maschinenteil ausgebildet sind, in der der Kolben bei einer Schwenkbewegung des Spannarms abrollt.

Der Reibungsdämpfer erzeugt durch Gleitbewegungen in dem Gleitkörper bei Schwenkbewegungen des Spannarms Reibung und dämpft damit den Zugmitteltrieb. Dabei sorgt die Feder dafür, dass der Gleitkörper und der Reibkörper stets an ihrer Berührfläche gegeneinander anliegen. Durch diese konusförmige Ausbildung von Reibkörper und Gleitkörper wird eine besonders bauraumsparende Dämpfungseinheit erreicht.

Zudem kann vorgesehen sein, dass die Dämpfungsvorrichtung einen beweglich in dem Aufnahmeraum gehalterten Zylinder umfasst, der an seiner von dem Maschinenteil abgewandten Stirnseite eine Abrundung aufweist, die an einer als eine Gegenfläche zu der Abrundung ausgebildeten Bodenfläche des Aufnahmeraumes anliegt, wobei eine Berührfläche zwischen der Abrundung und der Bodenfläche als Reibfläche nutzbar ist. Innerhalb des in dem Aufnahmeraum gehaltenen Zylinders der Dämpfungsvorrichtung können weitere reibend bzw. dämpfend wirkende Mittel angeordnet sein, beispielsweise solche gemäß dem oben beschrienen erfindungsgemäßen Reibungsdämpfer.

Bei dieser Ausführungsform ist vorzugsweise vorgesehen, dass die Versatzkompensationsmittel als eine kraftschlüssige Verbindung zwischen dem Kolben und dem Maschinenteil und als eine an der Dämpfungsvorrichtung angeordnete Halterung ausgebildet sind, wobei die Halterung die mit einem seitlichen Spiel in dem Aufnahmeraum angeordnete Dämpfungsvorrichtung gegen ein Herausfallen aus dem Spannarm sichert und eine Kompensationsbewegung der Dämpfungsvorrichtung zu einer Schwenkbewegung des Spannarms innerhalb des Aufnahmeraumes zulässt. Die Halterung kann vorteilhaft durch einen O-Ring gebildet sein. Diese Anordnung ist konstruktiv besonders einfach und kostengünstig.

Als Versatzkompensationsmittel sind aber auch ein Rollenlager oder eine Arre an dem Kolben und eine entsprechend dazu ausgeformte Gegenfläche an dem Maschinenteil möglich, an der sich das Rollenlager bzw. die Arre abstützt. Diese Kompensationsmittel ermöglichen eine besonders verschleißarme Bewegung zwischen dem Kolben und einer Kolbenabstützung an dem Maschinenteil.

Weiterhin kann vorgesehen sein, dass an dem Kolben ein sich zwischen dem Spannarm und dem Maschinenteil abstützendes Federelement angeordnet ist, das als ein eine Vorspannung des Zugmittels erzeugendes oder verstärkendes Spannmittel wirksam ist.

Über die Kolbenabstützung an dem Maschinenteil kann der an seiner Drehachse gelagerte Spannarm mit einer bestimmten Vorspannkraft auf das Zugmittel voreingestellt werden. Die Reibfläche kann zudem so ausgelegt sein, dass bei Schwenkbewegungen des Spannarms zunächst eine Haftreibung zu überwinden ist, die dann in eine Gleitreibung übergeht, wodurch die Vorspannung unterstützt wird. Durch die zusätzliche Feder ist eine weitere Möglichkeit gegeben, die Vorspannung zu unterstützten, wodurch die Vorspannung effizienter wird. Grundsätzlich ist es auch möglich, dass die zusätzliche Feder die gesamte notwendige Vorspannung erzeugt. Die erfindungsgemäße Dämpfungsvorrichtung kann somit als ein sehr effektives kombiniertes Spann- und Dämpfungssystem für den Zugmitteltrieb fungieren.

Schließlich kann noch vorgesehen sein, dass der Aufnahmeraum als ein Gehäuse ausgebildet ist, welches mit dem darin aufgenommenen Reibungsdämpfer ein vormontierbares Bauteil bildet, das in eine entsprechend ausgebildete Ausnehmung des Spannarms einfügbar ist. Das Gehäuse bildet mit dem Reibungsdämpfer eine komplette Dämpfungseinheit, die einfach in eine vorbereitete Ausnehmung des Spannarms einsteckbar und verankerbar ist. Dadurch wird der Montage- und Herstellungsaufwand, insbesondere in der Serienfertigung, verringert, was sich zeitsparend und kostengünstig auswirkt.

### Kurze Beschreibung der Zeichnungen

Die Erfindung wird im Folgenden anhand der beiliegenden Zeichnung an einigen Ausführungsformen näher erläutert. Darin zeigt
- Fig. 1: einen Spannarm eines Zugmitteltriebs im Ausriss mit einer ersten Ausführungsform einer erfindungsgemäßen Dämpfungsvorrichtung im Schnitt B-B von Fig. 2,
- Fig. 2: eine Draufsicht von oben auf den Spannarm,
- Fig. 3: eine zweite Ausführungsform der Dämpfungsvorrichtung in einem Längsschnitt des Spannarms im Ausriss,
- Fig. 4a: ein Rollenlager als eine Kolbenabstützung in einer Seitenansicht im Schnitt,
- Fig. 4b: das Rollenlager von Fig. 4a in einer Draufsicht von oben,
- Fig. 5: eine Arre als eine Kolbenabstützung in einer Seitenansicht im Schnitt, und
- Fig. 6: der Kolben der Ausführungsform von Fig. 1 mit einem zusätzlichen Federelement zur Erzeugung einer Vorspannung.

### Detaillierte Beschreibung der Zeichnungen

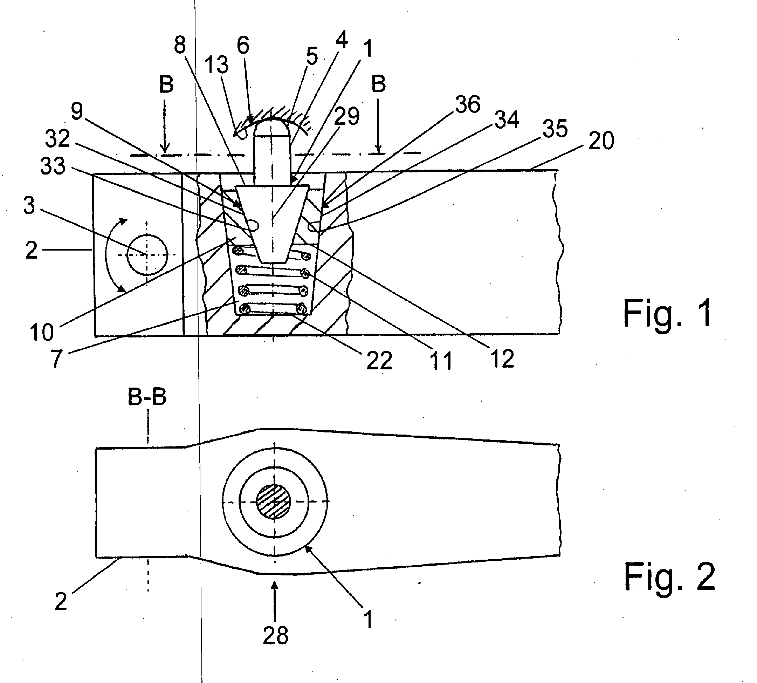

Die in Fig. 1 gezeigte Dämpfungsvorrichtung weist einen Reibungsdämpfer 1 auf, der in einen Spannarm (Hebelarm) 2 integriert ist. In der Draufsicht gemäß Fig. 2 ist erkennbar, dass der Spannarm 2 ein verbreitertes Profil 28 im Bereich des Reibungsdämpfers 1 aufweist. Der Spannarm 2 ist um einen Drehpunkt, bzw. eine Drehachse 3 drehbar gelagert und bildet eine Spannvorrichtung für ein Zugmittel in einem Zugmitteltrieb, bei dem sich der Spannarm 2 über eine (nicht dargestellte) Spannrolle an dem Zugmittel abstützt. Der Zugmitteltrieb kann beispielsweise zum Antrieb eines oder mehrere Nebenaggregate an einem Motor eines Kraftfahrzeuges angeordnet sein. Der Aufbau derartiger Zugmitteltriebe und deren Funktionsweise sind im Prinzip bekannt. Daher wird im Folgenden nur die erfindungsgemäße Dämpfungsvorrichtung im Detail beschrieben.

Der Reibungsdämpfer 1 ist quer beabstandet zu der Drehachse 3 in einen kegelförmigen Aufnahmeraum 7 eingesetzt. Er weist einen kegelförmigen Reibkörper 8 auf, der in einem als ein Kegelsitz ausgebildeten Gleitkörper 10 sitzt. Der Gleitkörper 10 ist vorteilhaft mit als Reiboberflächen ausgebildeten Wandungen (Innenwand 33, Außenwand 34) versehen. Die Außenwand 32 des Reibkörpers 8 kann als eine Reiboberfläche speziell bearbeitet sein.

Die konische Wand 35 des Aufnahmeraums 7 und die konische Außenwand 34 des Gleitkörpers 10 sind geometrisch so aufeinander abgestimmt, dass der Gleitkörper 10 in der Nähe zur seitlichen Außenwand 20 des Hebelarms 2 und beabstandet zu einer Bodenfläche 22 des Aufnahmeraums 7 beweglich und formschlüssig gehalten wird. Zwischen einer Unterseite 12 des Gleitkörpers 10 und dem Boden 22 des Aufnahmeraums 7 stützt sich ein Federelement 11 ab. Die Feder 11 ist vorteilhaft als eine konisch gewickelte Schraubendruckfeder ausgebildet, die den Kegelsitz bzw. Gleitkörper 10 gegen den Reibkörper 8 drückt. Der Reibkörper 8 stützt sich seinerseits über einen mit ihm vorteilhaft einstückig verbundenen, seitlich hervorstehenden Kolben 4 an einem Maschinenteil 6, beispielsweise einem Verbrennungsmotorgehäuse ab und drückt den Gleitkörper 10 gegen die Wand des Aufnahmeraumes 7.

Damit entstehen zwischen der Außenwand 32 des Reibkörpers 8 und der Innenwand 33 des Gleitkörpers 10 sowie zwischen der Außenwand 34 des Gleitkörpers 10 und der Innenwand 35 des Aufnahmeraums 7 stets in Kontakt befindliche gemeinsame Kontaktflächen 9 und 36, von denen wenigstens die innere Kontaktfläche 9 als eine gemeinsame Reibfläche fungiert.

Der Kolben 4 ist als ein zylinderförmiger Ansatz an dem Reibkörper 8 ausgebildet, dessen Stirnfläche 5 halbkugelförmig abgerundet ist. An dem Maschinenteil 6 ist benachbart zu der Stirnfläche 5 des Kolbens 4 eine Gegenfläche 13 ausgeformt, gegen die die abgerundete Kolbenstirnfläche 5 läuft und durch Abrollbewegungen den entstehenden Versatz bei der Schwenkbewegung des Spannarms 2 kompensiert. Durch diese Versatzkompensation wird sichergestellt, dass die Krafteinleitung stets senkrecht zur Kolbenstirnfläche 5, also in Richtung einer Längsachse 29 des Reibungsdämpfers 1, erfolgt und damit die erwünschten Gleitbewegungen innerhalb des Dämpfers 1 erzeugt werden, ohne dass in dem Aufnahmeraum 7 Klemmkräfte zwischen dem Reibkörper 8 und dem Gleitkörper 10 entstehen können.

Auf die im Betrieb des Zugmitteltriebs auf den Spannarm 2 übertragenen Schwingungen und Stöße reagiert der Spannarm 2 mit entsprechenden Schwenkbewegungen um die Drehachse 3. Diese Schwenkbewegungen führen zu Gleitbewegungen in Richtung der Längsachse 29 in dem konusförmigen Reibungsdämpfer 1 zwischen den Reibflächen 9 bzw. 36. Die dabei entstehende Reibung wirkt den Schwenkbewegungen entgegen und dämpft diese. Die Dämpfung wird über den Spannarm 2 und das Zugmittel auf den Zugmitteltrieb übertragen, der damit schwingungs- bzw. stoßgedämpft läuft.

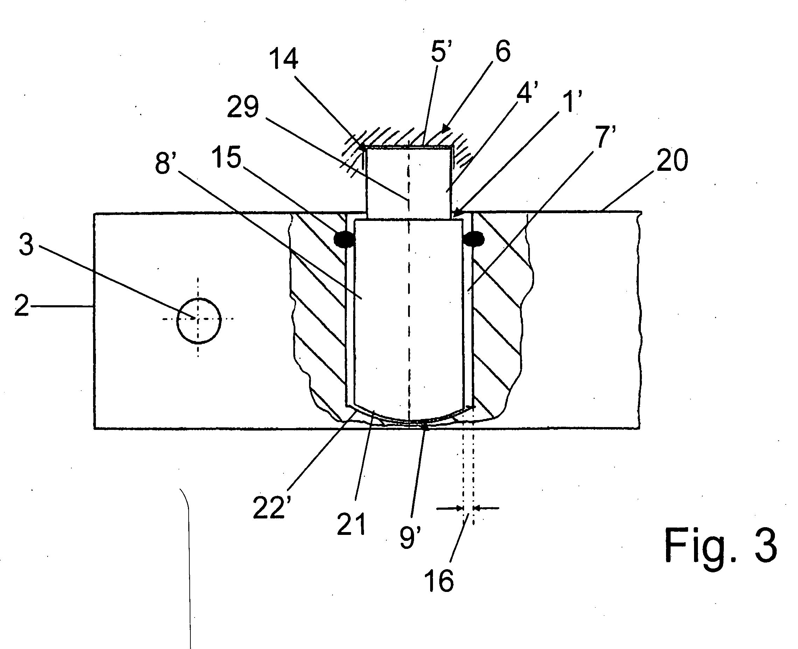

Fig. 3 zeigt eine Dämpfungsvorrichtung 8' mit einem zylinderförmigen Reibungsdämpfer 1'. Die Dämpfungsvorrichtung 8' ist einstückig mit einem zylindrischen Kolben 4' verbunden, der aus dem Spannarm 2 seitlich heraussteht. Die Dämpfungsvorrichtung 8' ist zudem über eine Halterung 15, vorteilhaft als O-Ring 15 ausgebildet, mit einem seitlichen Spiel 16 in einem Aufnahmeraum 7' innerhalb des Spannarms 2 gelagert. Der O-Ring 15 sichert den Reibungsdämpfer 1' bzw. die Dämpfungsvorrichtung 8' gegen Herausfallen und ermöglicht seitliche Schwenkbewegungen innerhalb des Aufnahmeraumes 7'.

Der Kolben 4' weist maschinenseitig eine gerade Stirnfläche 5' auf, die über ein kraftschlüssige Verbindung 14 mit dem Maschinenteil 6 verbunden ist. Der Kolben 4' ragt dazu vorteilhaft an seinem Ende in eine rechteckige Vertiefung an dem Maschinenteil 6. An der von dem Maschinenteil 6 abgewandten Stirnseite der Dämpfungsvorrichtung 8' ist ein Abrundung 21 vorgesehen, die zu einem Boden 22' des Aufnahmeraums 7' benachbart ist. Der Boden 22' ist zu der Abrundung 21 entsprechend ausgerundet, so dass sich eine Berührfläche 9' als Reibfläche ergibt.

Im Betrieb weicht der Reibungsdämpfer 1' Schwenkbewegungen des Spannarms 2 seitlich aus, wobei der Reibkörper 8' über die Reibfläche 9' gleitet und dabei die Schwenkbewegungen dämpft. Zusätzlich können innerhalb der Dämpfungsvorrichtung 8' Reibdämpfungsmittel gemäß der Figur 1 angeordnet sein.

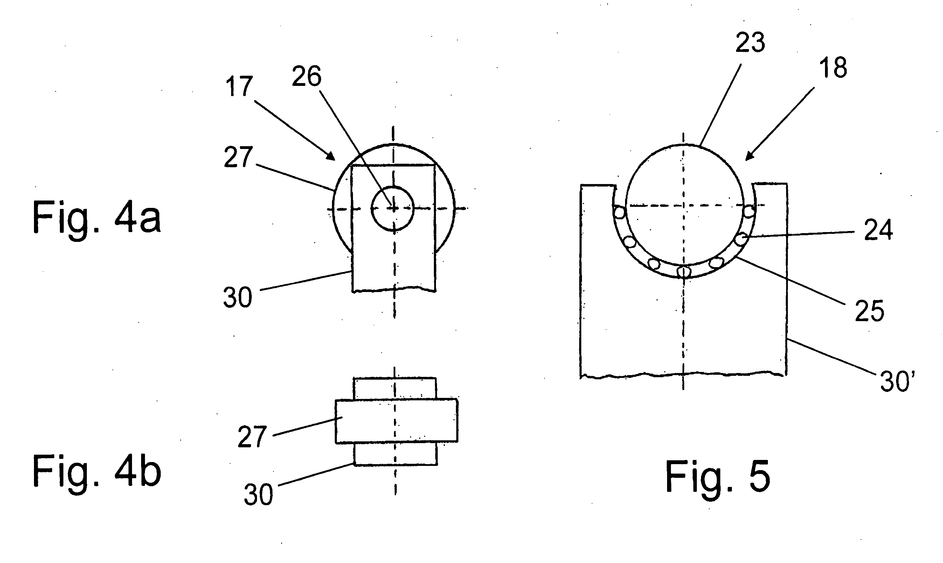

Die Figuren 4a und 4b zeigen ein Rollenlager 17, über das die Kompensationsbewegungen zwischen dem Heibungsdämpfer 1 und dem Masechinenteil 6 ausgleichbar sind. Das Rollenlager 17 weist eine Rolle 27 auf, die an einem Drehlager 26 eines Verbindungsstücks 30 zu dem Kolben 4 bzw. dem Reibungsdämpfer 1 befestigt ist. Die Rolle 27 stützt sich dabei an dem Maschinenteil 6 ab.

Fig. 5 zeigt eine weitere Möglichkeit, eine Bewegung zwischen dem Kolben 4 und der Kolbenabstützung an dem Maschinenteil 6 zu erlauben. Dabei ist eine Arre 18 mit einer Rolle 23 vorgesehen, die in einer halbkreisförmigen Ausnehmung 25 eines Verbindungsstücks 30' zu dem Kolben 4 mit einem Kugellager 24 gelagert ist.

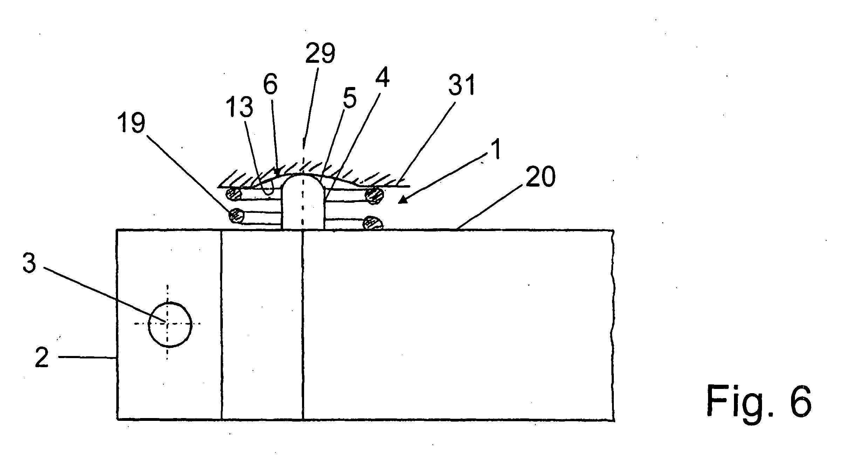

Die Fig. 6 zeigt schließlich eine Dämpfungsvorrichtung, bei der eine Vorspannfeder 19 vorgesehen ist. Zur Vereinfachung sind hier nur die aus dem Spannarm 2 herausstehenden Details gezeigt. Die Feder 19 ist vorteilhaft als eine zylindrische Schraubendruckfeder ausgebildet. Sie umfasst den Kolben 4 mit der abgerundeten Stirnfläche 5 koaxial und stützt sich zwischen der Außenwand 20 des Spannarms 2 und einer Umrandung 31 der Gegenfläche 13 an dem Maschinenteil 6 ab.

Bei Schwenkbewegungen des Spannarms 2 rollt die Abrundung 5 auf der Fläche 13 ab. Dabei wird die Feder 19 verkippt und gestaucht bzw. gedehnt. Die Federkraft wirkt dieser Bewegung entgegen, wodurch eine Spannkraft über den Spannarm 2 auf das Zugmittel übertragen wird. Die Feder 19 kann mit einer Vorspannung zwischen dem Arm 2 und dem Maschinenteil 6 eingeklemmt sein, so dass sie über die Drehachse 3 des Spannarms 2 eine permanente Vorspannung des Zugmittels bewirkt, die bei einer entsprechenden Auslegung die gesamte nötige Vorspannung zur Sicherstellung eines Reibschlusses zwischen dem Zugmittel und einer (nicht dargestellten) Riemenscheibe, auf dem das Zugmittel abläuft, zur Verfügung stellt, so dass die Dämpfungsvorrichtung gleichzeitig als eine Zugmittelvorspanneinheit und als eine Dämpfungseinheit fungiert.

### Bezugszeichenliste

- 1: Reibungsdämpfer
- 2: Spannarm
- 3: Drehachse
- 4, 4': Kolben
- 5, 5': Kolbenstirnfläche
- 6: Maschinenteil
- 7, 7': Aufnahmeraum
- 8: Reibkörper
- 8': Dämpfungsvorrichtung
- 9, 9': Reibfläche
- 10: Gleitkörper
- 11: Federelement
- 12: Unterseite
- 13: Gegenfläche
- 14: Verbindung
- 15: Halterung
- 16: Spiel
- 17: Rollenlager
- 18: Arre
- 19: Federelement
- 20: Seitenwand
- 21: Abrundung
- 22,22': Boden
- 23: Rolle
- 24: Kugellager
- 25: Ausnehmung
- 26: Drehlager
- 27: Rolle
- 28: Profilverbreiterung
- 29: Längsachse
- 30, 30': Verbindungsstück
- 31: Umrandung
- 32: Außenwand Reibkörper
- 33: Innenwand Gleitkörper
- 34: Außenwand Gleitkörper
- 35: Wandung Aufnahmeraum
- 36: Kontaktfläche

## Patentansprüche

1. Dämpfungsvorrichtung für einen Zugmitteltrieb, bei der sich ein um eine Drehachse (3) schwenkbarer Spannarm (2) zur Erzeugung und Aufrechterhaltung einer Vorspannung eines Zugmittels über eine Spannrolle an dem Zugmittel mit Hilfe von mechanischen Dämpfungsmitteln dämpfend abstützt, **dadurch gekennzeichnet, dass** in den Spannarm (2) ein quer zur Drehachse (3) beabstandeter Reibungsdämpfer (1, 1') integriert ist, mit einem seitlich aus dem Spannarm (2) vorstehenden Kolben (4, 4'), dessen Ende (5, 5') sich stirnseitig an einem ortsfesten Maschinenteil (6) abstützt, mit Mitteln zur Kompensation eines durch Schwenkbewegungen des Spannarms (2) entstehenden Versatzes zwischen dem Maschinenteil (6) und dem Spannarm (2), und mit einem in einem Aufnahmeraum (7, 7') innerhalb des Spannarms (2) angeordneten Reibkörper (8, 8'), über den Schwenkbewegungen des Spannarms (2) durch eine damit verbundene Gleitreibung zwischen dem Reibkörper (8, 8') und einer Reibfläche (9, 9') dämpfbar sind.

2. Dämpfungsvorrichtung nach Anspruch 1, **dadurch gekennzeichnet, dass** der Reibkörper (8) als ein mit seiner Grundfläche in Richtung des Maschinenteils (6) ausgerichteter Kegelstumpf ausgebildet ist, der in einem in dem Aufnahmeraum (7) gehaltenen und als ein Gleitkörper (10) ausgebildeten ringförmigen Kegelsitz sitzt, der mit Hilfe eines Federelementes (11) formschlüssig gegen den Kegelstumpf (8) gehalten wird, wobei eine Berührfläche zwischen dem Kegelstumpf (8) und dem Kegelsitz (10) die Reibfläche (9) bildet.

3. Dämpfungsvorrichtung nach Anspruch 1 oder 2, **dadurch gekennzeichnet, dass** die Dämpfungsvorrichtung (8') einen beweglich in dem Aufnahmeraum (7') gehalterten Zylinder umfasst, der an seiner von dem Maschinenteil (6) abgewandten Stirnseite eine Abrundung (21) aufweist, die an einer als eine Gegenfläche zu der Abrundung (21) ausgebildeten Bodenfläche (22') des Aufnahmeraumes (7') anliegt, wobei eine Berührfläche zwischen der Abrundung (21) und der Bodenfläche (22') als Reibfläche (9') nutzbar ist.

4. Dämpfungsvorrichtung nach einem der Ansprüche 1 bis 2, **dadurch gekennzeichnet, dass** die Versatzkompensationsmittel als eine Abrundung an der zur dem Maschinenteil (6) benachbarten Stirnfläche (5) des Kolbens (4) und als eine zu der abgerundeten Stirnfläche (5) als eine Gegenfläche (13) ausgebildete Kontaktfläche an dem Maschinenteil (6), in der der Kolben (4) bei einer Schwenkbewegung des Spannarms (2) abrollt, ausgebildet sind.

5. Dämpfungsvorrichtung nach Anspruche 3, **dadurch gekennzeichnet, dass** die Versatzkompensationsmittel als eine kraftschlüssige Verbindung (14) zwischen dem Kolben (4') und dem Maschinenteil (6) und als eine an der Dämpfungsvorrichtung (8') angeordnete Halterung (15) ausgebildet sind, wobei die Halterung (15) die mit einem seitlichen Spiel (16) in dem Aufnahmeraum (7') angeordnete Dämpfungsvorrichtung (8') gegen ein Herausfallen aus dem Spannarm (2) sichert und eine Kompensationsbewegund der Dämpfungsvorrichtung (8') zu einer Schwenkbewegung des Spannarms (2) innerhalb des Aufnahmeraumes (7') zulässt.

6. Dämpfungsvorrichtung nach Anspruch 5, **dadurch gekennzeichnet, dass** die Kolbenhalterung (15) als ein O-Ring ausgebildet ist.

7. Dämpfungsvorrichtung nach einem der Ansprüche 1 bis 3, **dadurch gekennzeichnet, dass** die Versatzkompensationsmittel als ein Rollenlager (17) an dem Kolben (4) und als eine Gegenfläche zu dem Rollenlager (17) an dem Maschinenteil (6) ausgebildet sind, wobei sich das Rollenlager (17) an der Gegenfläche abstützt.

8. Dämpfungsvorrichtung nach einem der Ansprüche 1 bis 3, **dadurch gekennzeichnet, dass** die Versatzkompensationsmittel als eine Arre (18) an dem Kolben (4) und als eine Gegenfläche zu der Arre (18) an dem Maschinenteil (6) ausgebildet sind, wobei sich die Arre (18) an der Gegenfläche abstützt.

9. Dämpfungsvorrichtung nach einem der Ansprüche 1 bis 8, **dadurch gekennzeichnet, dass** an dem Kolben (4) ein sich zwischen dem Spannarm (2) und dem Maschinenteil (6) abstützendes Federelement (19) angeordnet ist, das als ein eine Vorspannung des Zugmittels erzeugendes oder verstärkendes Spannmittel wirksam ist.

10. Dämpfungsvorrichtung nach einem der Ansprüche 1 bis 9, **dadurch gekennzeichnet, dass** der Aufnahmeraum (7, 7') als ein Gehäuse ausgebildet ist, das mit dem darin aufgenommenen Reibungsdämpfer (1, 1') ein vormontierbares Bauteil bildet, das in eine entsprechend ausgebildete Ausnehmung des Spannarms (2) einfügbar ist.

## Claims

1. Damping device for a traction mechanism drive, in which damping device a tensioning arm (2), which is pivotable about an axis of rotation (3), for generating and maintaining a preload of a traction mechanism is supported on the traction mechanism via a tensioning roller in a manner damped by mechanical damping means, **characterized in that** a friction damper (1, 1') which is spaced apart from and perpendicular to the axis of rotation (3) is integrated into the tensioning arm (2), said friction damper having a piston (4, 4') which protrudes laterally out of the tensioning arm (2) and the end of which (5, 5') is supported at the end side against a positionally fixed machine part (6), having means for compensating an offset, arising as a result of pivoting movements of the tensioning arm (2), between the machine part (6) and the tensioning arm (2), and having a friction body (8, 8') which is arranged in a receiving space (7, 7') within the tensioning arm (2) and by means of which pivoting movements of the tensioning arm (2) can be damped by means of associated sliding friction between the friction body (8, 8') and a friction surface (9, 9').

2. Damping device according to Claim 1, **characterized in that** the friction body (8) is designed as a truncated cone which is aligned with its base surface in the direction of the machine part (6) and which is seated in an annular conical seat held in the receiving space (7) and designed as a sliding body (10), which conical seat is held in a positively locking manner against the truncated cone (8) by means of a spring element (11), wherein a contact area between the truncated cone (8) and the conical seat (10) forms the friction surface (9).

3. Damping device according to Claim 1 or 2, **characterized in that** the damping device (8') comprises a cylinder which is retained in a movable manner in the receiving space (7') and which, at its end side facing away from the machine part (6), has a rounded portion (21) which bears against a bottom surface (22'), which is formed as a counterpart surface to the rounded portion (21), of the receiving chamber (7'), wherein a contact area between the rounded portion (21) and the bottom surface (22') can be utilized as a friction surface (9').

4. Damping device according to either of Claims 1 and 2, **characterized in that** the offset compensation means are designed as a rounded portion on that end surface (5) of the piston (4) which is adjacent to the machine part (6) and as a contact surface, formed as a counterpart surface (13) to the rounded end surface (5), on the machine part (6), in which counterpart surface the piston (4) rolls during a pivoting movement of the tensioning arm (2).

5. Damping device according to Claim 3, **characterized in that** the offset compensation means are designed as a non-positively locking connection (14) between the piston (4') and the machine part (6) and as a retainer (15) arranged on the damping device (8'), wherein the retainer (15) secures the damping device (8'), which is arranged with lateral play (16) in the receiving space (7'), so as to prevent it from falling out of the tensioning arm (2) and allows the damping device (8') to perform within the receiving space (7') a compensation movement in relation to a pivoting movement of the tensioning arm (2).

6. Damping device according to Claim 5, **characterized in that** the piston retainer (15) is formed as an O-ring.

7. Damping device according to one of Claims 1 to 3, **characterized in that** the offset compensation means are designed as a roller bearing (17) on the piston (4) and as a counterpart surface to the roller bearing (17) on the machine part (6), wherein the roller bearing (17) is supported on the counterpart surface.

8. Damping device according to one of Claims 1 to 3, **characterized in that** the offset compensation means are designed as a detent (18) on the piston (4) and as a counterpart surface to the detent (18) on the machine part (6), wherein the detent (18) is supported on the counterpart surface.

9. Damping device according to one of Claims 1 to 8, **characterized in that** a spring element (19) which is supported between the tensioning arm (2) and the machine part (6) is arranged on the piston (4), which spring element (19) acts as a tensioning means which generates or boosts a preload of the traction mechanism.

10. Damping device according to one of Claims 1 to 9, **characterized in that** the receiving space (7, 7') is designed as a housing which, with the friction damper (1, 1') held therein, forms a preassemblable component which can be inserted into a correspondingly designed recess of the tensioning arm (2).

## Revendications

1. Dispositif d'amortissement pour un entraînement à moyen de traction, dans lequel un bras de tensionnement (2) pouvant pivoter autour d'un axe de rotation (3) pour produire et maintenir une précontrainte d'un moyen de traction s'appuie en effectuant un effet d'amortissement par le biais d'une poulie de tensionnement sur le moyen de traction à l'aide de moyens d'amortissement mécaniques, **caractérisé en ce qu'**un amortisseur de friction (1, 1') espacé transversalement à l'axe de rotation (3) est intégré dans le bras de tensionnement (2), avec un piston (4, 4') saillant latéralement hors du bras de tensionnement (2), dont l'extrémité (5, 5') s'appuie du côté frontal contre une partie de machine fixe (6), avec des moyens pour compenser un décalage produit par des mouvements de pivotement du bras de tensionnement (2) entre la partie de machine (6) et le bras de tensionnement (2), et avec un corps de friction (8, 8') disposé dans un espace de réception (7, 7') à l'intérieur du bras de tensionnement (2), par le biais duquel des mouvements de pivotement du bras de tensionnement (2) peuvent être amortis par un frottement de glissement associé entre le corps de friction (8, 8') et une surface de friction (9, 9').

2. Dispositif d'amortissement selon la revendication 1, **caractérisé en ce que** le corps de friction (8) est réalisé sous la forme d'un tronc de cône orienté avec sa surface de base dans la direction de la partie de machine (6), qui repose dans un siège conique de forme annulaire maintenu dans l'espace de réception (7) et réalisé sous forme de corps glissant (10), qui est maintenu à l'aide d'un élément de ressort (11) par engagement positif contre le tronc de cône (8), une surface de contact entre le tronc de cône (8) et le siège de cône (10) formant la surface de friction (9).

3. Dispositif d'amortissement selon la revendication 1 ou 2, **caractérisé en ce que** le dispositif d'amortissement (8') comprend un cylindre maintenu mobile dans l'espace de réception (7'), qui présente sur son côté frontal opposé à la partie de machine (6) un arrondi (21), qui s'applique contre une surface de fond (22') de l'espace de réception (7') réalisée sous forme de surface conjuguée à l'arrondi (21), une surface de contact entre l'arrondi (21) et la surface de fond (22') pouvant être utilisée comme surface de friction (9').

4. Dispositif d'amortissement selon l'une quelconque des revendications 1 à 2, **caractérisé en ce que** les moyens de compensation du décalage sont réalisés sous forme d'arrondi au niveau de la surface frontale (5) adjacente à la partie de machine (6) du piston (4) et sous forme d'une surface de contact réalisée sous forme de surface conjuguée (13) par rapport à la surface frontale arrondie (5), sur la partie de machine (6), dans laquelle le piston (4) roule lors d'un mouvement de pivotement du bras de tensionnement (2).

5. Dispositif d'amortissement selon la revendication 3, **caractérisé en ce que** les moyens de compensation du décalage sont réalisés sous forme de connexion par engagement par force (14) entre le piston (4') et la partie de machine (6) et sous forme d'une fixation (15) disposée sur le dispositif d'amortissement (8'), la fixation (15) fixant le dispositif d'amortissement (8') disposé avec un jeu latéral (16) dans l'espace de réception (7') pour l'empêcher de tomber du bras de tensionnement (2), et permettant un mouvement de compensation du dispositif d'amortissement (8') par rapport à un mouvement de pivotement du bras de tensionnement (2) à l'intérieur de l'espace de réception (7').

6. Dispositif d'amortissement selon la revendication 5, **caractérisé en ce que** la fixation de piston (15) est réalisée sous forme de joint torique.

7. Dispositif d'amortissement selon l'une quelconque des revendications 1 à 3, **caractérisé en ce que** les moyens de compensation du décalage sont réalisés sous forme de palier à rouleaux (17) sur le piston (4) et sous forme de surface conjuguée au palier à rouleaux (17) sur la partie de machine (6), le palier à rouleaux (17) s'appuyant sur la surface conjuguée.

8. Dispositif d'amortissement selon l'une quelconque des revendications 1 à 3, **caractérisé en ce que** les moyens de compensation du décalage sont réalisés sous forme de butée (18) sur le piston (4) et sous forme de surface conjuguée à la butée (18) sur la partie de machine (6), la butée (18) s'appuyant sur la surface conjuguée.

9. Dispositif d'amortissement selon l'une quelconque des revendications 1 à 8, **caractérisé en ce qu'**un élément de ressort (19) s'appuyant entre le bras de tensionnement (2) et la partie de machine (6) est disposé sur le piston (4), lequel élément de ressort agit en tant que moyen de tensionnement produisant ou renforçant une précontrainte du moyen de traction.

10. Dispositif d'amortissement selon l'une quelconque des revendications 1 à 9, **caractérisé en ce que** l'espace de réception (7, 7') est réalisé sous forme de boîtier qui forme avec l'amortisseur de friction (1, 1') reçu à l'intérieur un composant prémontable, qui peut être introduit dans un évidement du bras de tensionnement (2) réalisé de manière correspondante.
